# EUROPEAN PATENT SPECIFICATION

(11) **EP 2 477 636 B1**
(45) Date of publication and mention of the grant of the patent: **06.05.2015**
(21) Application number: 10754751.5
(22) Date of filing: 17.09.2010
(51) Int. Cl.: A61K 39/00, A61P 9/10

(54) **Tolerogenic dendritic cells for the treatment and prevention of atherosclerosis**
Tolerogene dendritische Zellen zur Behandlung und Vorbeugung von Atherosklerose
Cellules dendritiques tolérogéniques pour le traitement et la prévention de l'athérosclérose

(30) Priority: 18.09.2009 EP 09170746
(43) Date of publication of application: 25.07.2012
(73) Proprietor: CardioVax, LLC, Torrance, CA 90501 (US)
(72) Inventor: HANSSON, Göran, S-112 39 Stockholm (SE); HERMANSSON, Andreas, S-17278 Sundbyberg (SE)
(74) Representative: Copsey, Timothy Graham
(86) International application number: PCT/EP2010/063743
(87) International publication number: WO 2011/033090

(56) References cited:
- WO-A1-2008/055354
- WO-A2-01/23414
- WO-A2-99/31227
- WO-A2-2007/116409
- US-A1- 2003 165 819
- US-A1- 2006 018 929
- DATABASE CA [Online] CHEMICAL ABSTRACTS SERVICE, COLUMBUS, OHIO, US; 14 January 2008 (2008-01-14), LI, DAZHU ET AL: "Effects of rapamycin-treated HSP60-pulsed dendritic cells on progression of atherosclerotic plaque in mice" XP002563749 retrieved from STN Database accession no. 2008:50389 & ZHONGGUO BINGLI SHENGLI ZAZHI , 22(6), 1079-1082 CODEN: ZBSZEB; ISSN: 1000-4718, 2006,
- YANG ET AL: "Generation of HSP60-specific regulatory T cell and effect on atherosclerosis" CELLULAR IMMUNOLOGY, ACADEMIC PRESS, SAN DIEGO, CA, US LNKD- DOI:10.1016/J.CELLIMM.2007.01.002, vol. 243, no. 2, 21 March 2007 (2007-03-21), pages 90-95, XP005927483 ISSN: 0008-8749
- DATABASE BIOSIS [Online] BIOSCIENCES INFORMATION SERVICE, PHILADELPHIA, PA, US; October 2007 (2007-10), HERBIN OLIVIER ET AL: "Continuous subcutaneous delivery of apolipoprotein B-derived peptides induces T cells anergy and reduces the progression of established atherosclerosis in mice" XP002563751 Database accession no. PREV200800197647 & CIRCULATION, vol. 116, no. 16, Suppl. S, October 2007 (2007-10), page 145, 80TH ANNUAL SCIENTIFIC SESSION OF THE AMERICAN-HEART-ASSOCIATION; ORLANDO, FL, USA; NOVEMBER 04 -07, 2007 ISSN: 0009-7322

## Description

### Field

The present disclosure relates to tolerogenic dendritic cells and related uses, compositions, methods and systems, with particular reference to treatment and/or prevention of atherosclerosis.

### Background

Atherosclerosis is currently viewed as a chronic lipid-related and immune-mediated inflammatory disease of the arterial walls. Many immune components have been identified that participate in atherogenesis and preclinical studies have yielded promising results suggesting that immuno-modulatory therapies targeting these components can reduce atherosclerosis

### Summary

Provided herein are tolerogenic dendritic cells and related compositions, methods and systems that, in various embodiments, are suitable to treat or prevent atherosclerosis and/or a condition associated thereto in an individual.

One aspect of the present disclosure relates to an antigen specific tolerogenic antigen-presenting cell, which presents an antigenic portion of at least one autoantigen associated to atherogenesis and/or atherosclerosis in an individual, a fragment thereof or a derivative thereof, the antigen-specific tolerogenic antigen-presenting cells being specific for the at least one autoantigen fragment thereof or derivative thereof.

Another aspect of the present disclosure relates to an antigen-specific tolerogenic antigen-presenting cell, which is obtainable by incubating at least one autoantigen associated to atherogenesis and/or atherosclerosis in an individual, a fragment thereof, a derivative thereof or a precursor thereof, with an antigen-presenting cell treated with an immunosuppressive cytokine before during and/or after the incubating.

Another aspect of the present disclosure relates to an antigen-specific tolerogenic antigen-presenting cells presenting at least one antigenic portion of an autoantigen associated to atherogenesis in an individual, a fragment thereof or a derivative thereof, for use in medicine, and in particular for treatment and/or prevention of atherosclerosis and/or a condition associated thereto.

Another aspect of the disclosure is a method for treatment and/or prevention of atherosclerosis or a condition associated thereto in an individual, the method comprising administering an antigen-specific tolerogenic antigen-presenting cell described herein to the individual.

Another aspect of the disclosure is a composition comprising an antigen-specific tolerogenic antigen-presenting cell herein described together with a suitable vehicle. In some embodiments, the composition can be a pharmaceutical composition, intended for use in treatment of atherosclerosis or of a condition associated thereto.

The methods and systems herein described can be used in connection with applications wherein a tolerogenic antigen-presenting cell and/or a therapeutic or preventive effect for atherosclerosis in an individual is desired.

The invention is as defined in the claims

The details of one or more embodiments of the disclosure are set forth in the accompanying drawings and the description below. Other features, objects, and advantages will be apparent from the description and drawings, and from the claims.

### Brief description of the drawings

The accompanying drawings, which are incorporated into and constitute a part of this disclosure, illustrate one or more embodiments of the present disclosure and, together with the detailed description and examples sections, serve to explain the principles and implementations of the tolerogenic dendritic cells and related applications herein described.

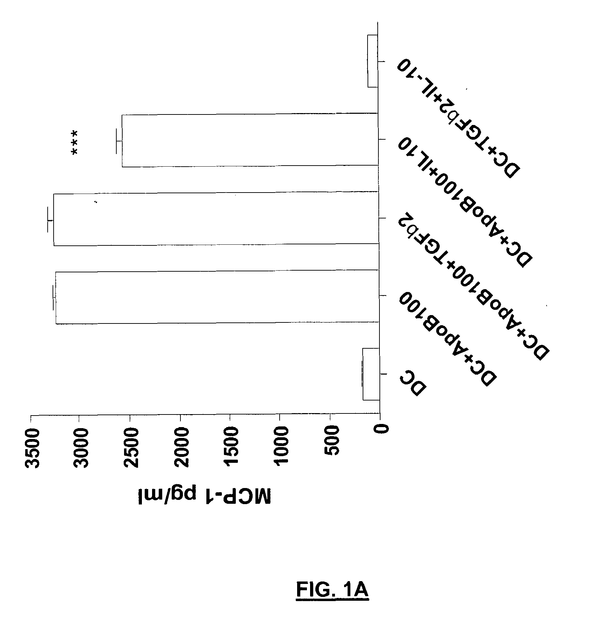

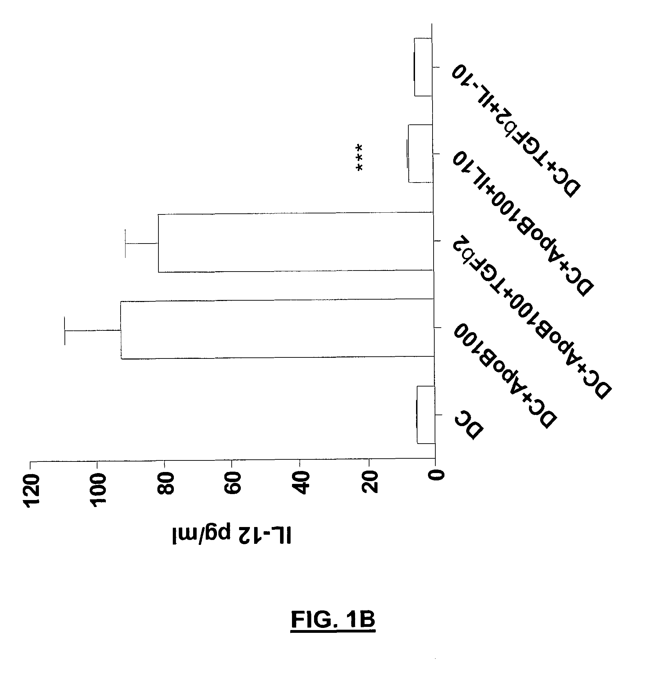

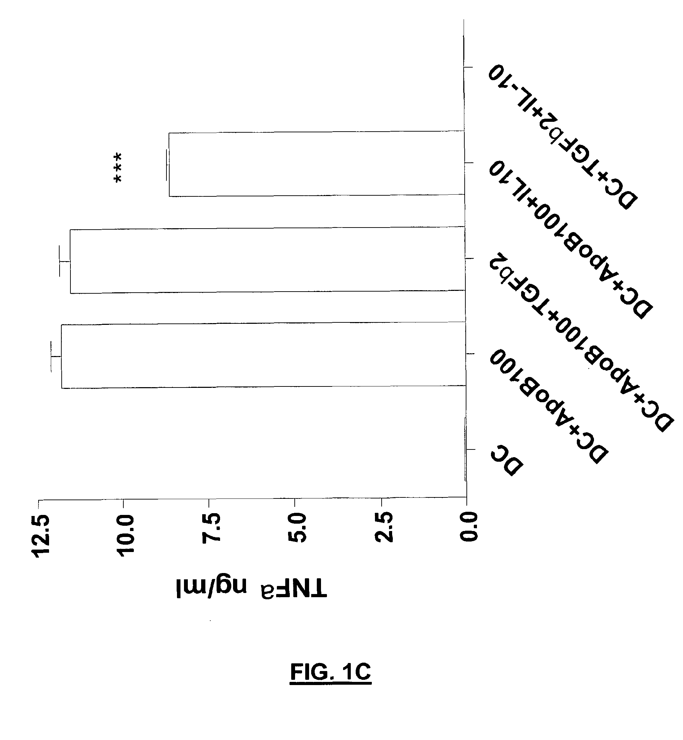

**Figure 1** shows charts illustrating an impaired capability of tolerogenic dendritic cells (DC) to induce T cell effector responses in vitro according to an embodiment herein described. In particular, **Figure 1A** describes a diagram illustrating levels of chemokine monocyte chemotactic protein-1 (MCP-1) produced by DC cells alone or pretreated with ApoB100, IL10 and/or TGFβ2 as indicated. **Figure 1B** describes a chart illustrating levels of IL-12 produced by DC cells alone or pretreated with ApoB100, IL10 and/or TGFβ2 as indicated. **Figure** 1C describes a chart illustrating levels of cytokine tumor necrosis factor-α (TNF- α) produced by DC cells alone or pretreated with ApoB100, IL10 and/or TGFβ2 as indicated. Data marked with *** indicate p<0.001, as compared to DC + ApoB100 (n = 6).

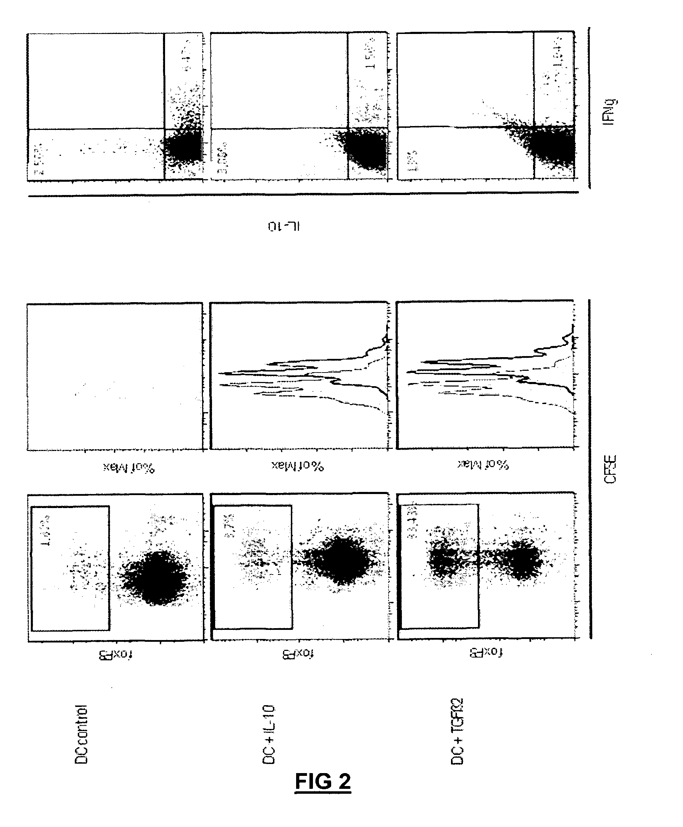

**Figure 2** shows charts illustrating the ability of tolerogenic DC to inhibit production of interferon-gamma and stimulate de novo generation of regulatory T cells according to an embodiment herein described and the ability of tolerogenic DC to dampen proliferation of activated T cells according to an embodiment herein described. The left column shows flow cytometric analysis of CD4⁺ T cells that were depleted of natural regulatory T cells, stained with CFSE to visualize cell proliferation, co-cultured for 96h and then stained with antibodies to Foxp3 alone or following treatment with IL-10 or TGF-b2 as indicated. The graphs show CFSE fluorescence on the x axis and Foxp3 on the y axis. The middle column shows in histogram format CFSE fluorescence of the samples displayed in the 2-dimensional plots of the left column. The right column shows intracellular staining of CD4+ T cells after 96h of co-incubation The flow cytometric plots show staining for IFNγ (x-axis) and IL-10 (y-axis); the number of cells positive for each cytokine are indicated. The three plots are from T cell cultures exposed to DC as described in the left margin of **Figure 2**

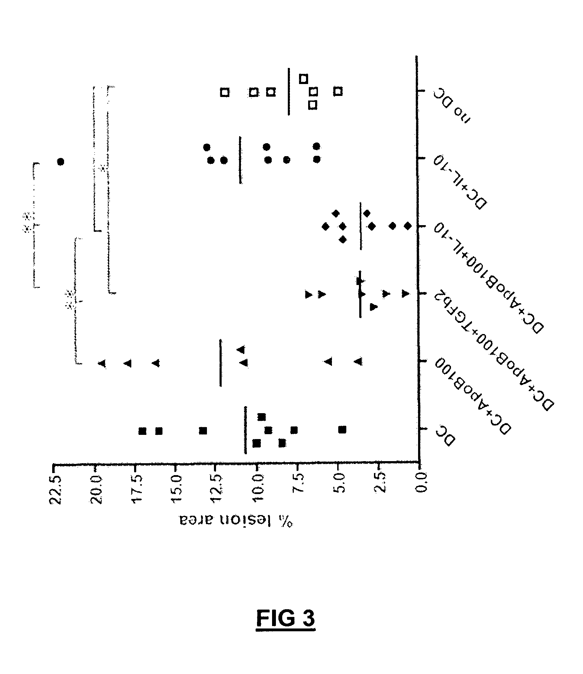

**Figure** 3 show a chart illustrating atherosclerotic lesion size in the proximal aorta in presence or absence of tolerogenic DC according to an embodiment herein described. In particular, **Figure 3** shows the area occupied by atherosclerotic lesions in the proximal aorta of of huApoB100^{tg}x*ldlr*-/- mice following treatment with DC alone or DC pretreated with ApoB100, IL10 and/or TGFβ2 as indicated. Data marked with ** indicate significantly different P<0.01. Data marked with * indicate significantly different at P<0.05. Brackets above asterisks indicate groups compared in each significance test. Each dot, box, triangle or diamond represents the lesion area in one individual mouse of each group, with mean values for each group indicate by the horizontal lines. Dissected aortas were stained with Sudan IV and % lesion area of total vessel area was calculated using computerized image analysis of the aortic surface.

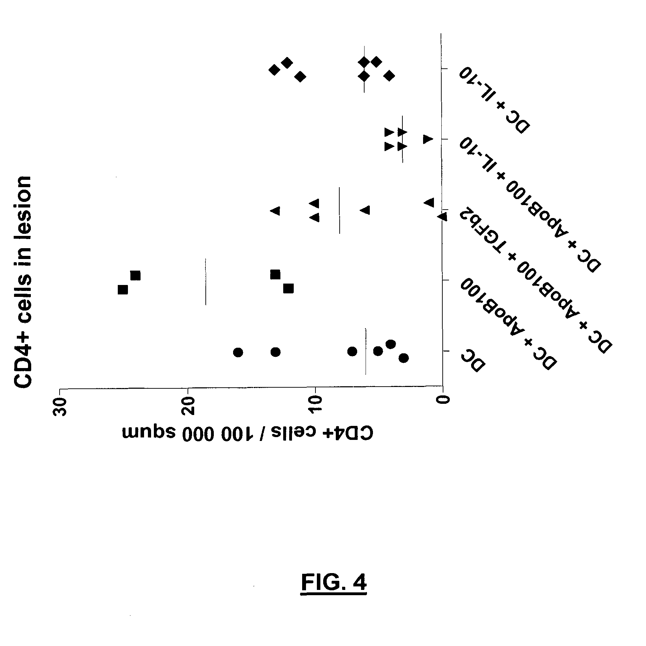

**Figure 4** shows a chart illustrating the effect of tolerogenic DC on infiltration of CD4+ T cells in aortic root lesions of huApoB100^{tg}x*ldlr*-/- mice according to an embodiment herein described. In particular, **Figure 4** shows the number of CD4+ T cells per 100,000 square micrometer (sqµm) lesion in histological cross-sections from the aortic root. CD4+ T cells were visualized by immunoperoxidase staining using anti-CD4 antibodies. Symbols are as described for **Figure 3****.** The different groups had undergone treatment with DC alone or DC pretreated with ApoB100, IL10 and/or TGFb2 as indicated.

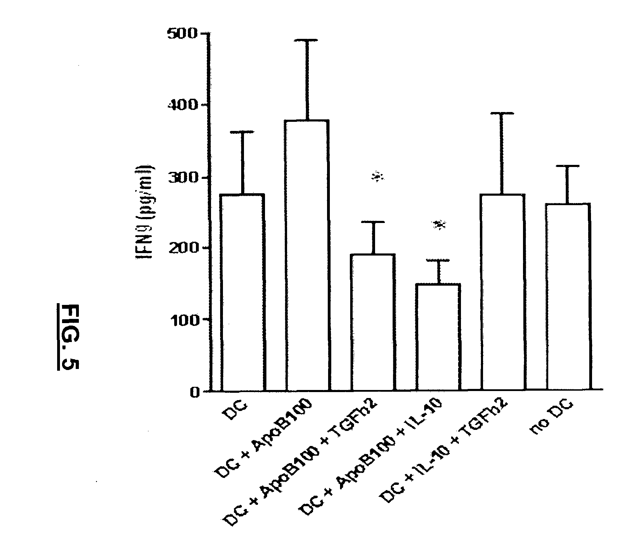

**Figure 5** shows a chart illustrating variations of interferon-gamma levels in plasma of mice after injection of ApoB100-specific tolerogenic DC according to an embodiment herein described. In particular, **Figure 5** shows the IFN γ concentration in plasma of mice following administration of DC alone or pretreated with ApoB100, IL10 and/or TGFb2 as indicated. Data marked with * indicate significant difference at P<0.05 from the group treated with DC + ApoB100.

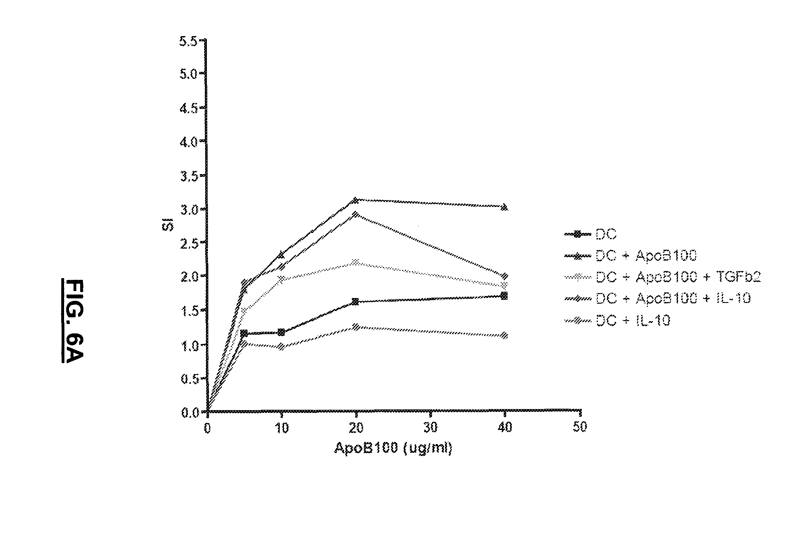

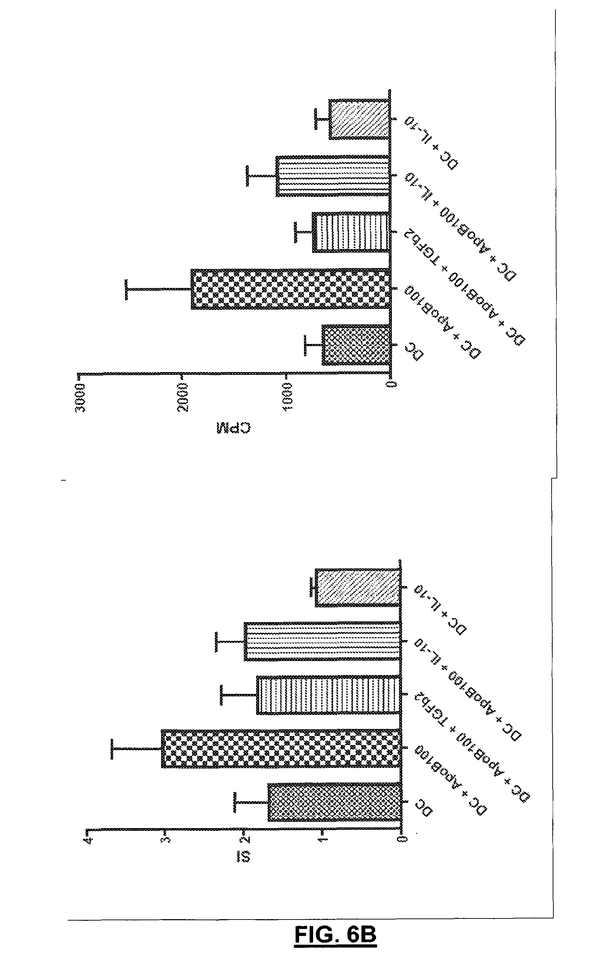

**Figure 6** shows charts illustrating T cell activation in response to ApoB100 in splenocytes of mice from the different treatment groups according to an embodiment herein described. In particular, T cell activation was measured as incorporation of ³H-thymidine into DNA and is shown as variation of the stimulation index (cpm of treated group - cpm for control / cpm for control: **Figure 6a** and **Figure 6b** **left graph),** and also as raw data (cpm) **(****Figure 6b** **right graph)** following administration of DC alone or pretreated with ApoB100, IL10 and/or TGFb2 as indicated.

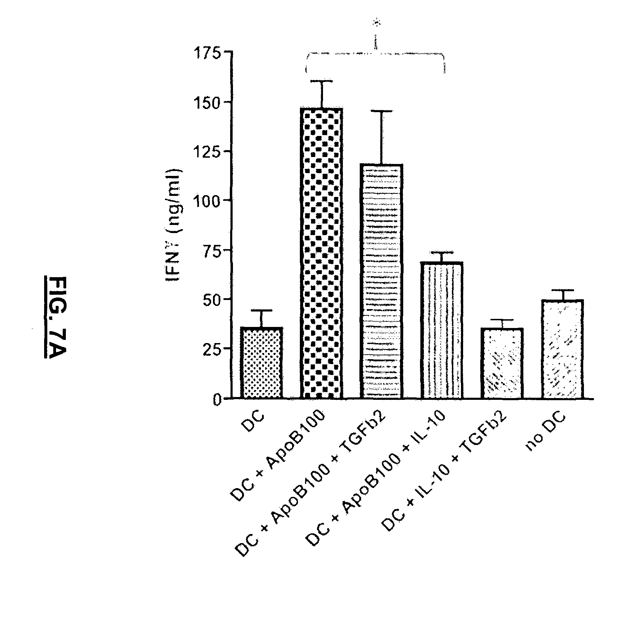

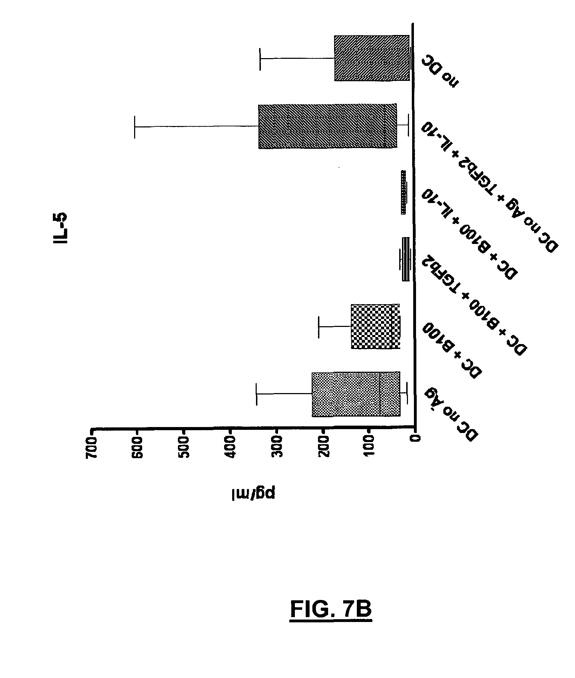

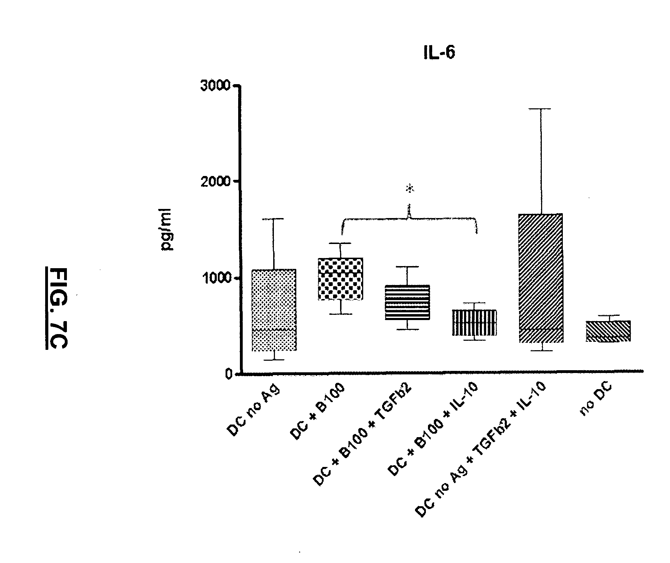

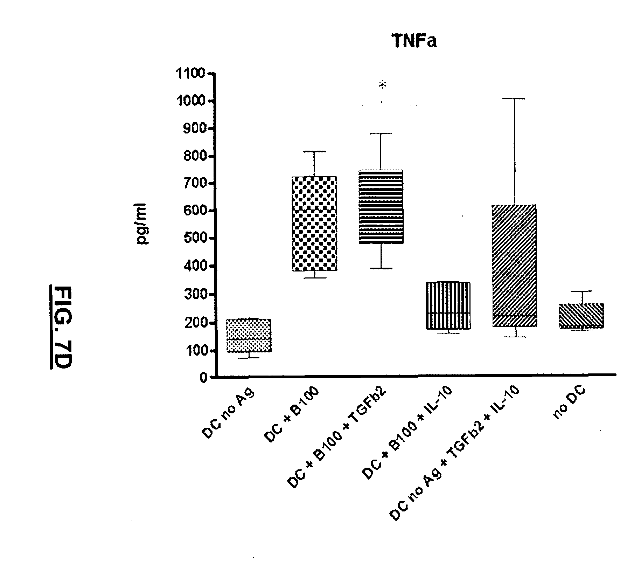

**Figure 7** shows charts illustrating cytokine secretion in response to ApoB100 in splenocytes from mice undergoing different treatments according to an embodiment herein described. One week after DC treatment, mice were immunized subcutaneously with ApoB100 protein to boost the immune response to ApoB100. * indicates P<0.05, with brackets showing the groups compared in the significance tests. Interferon-gamma **(****Figure 7a****),** interleukin-5 **(****Figure 7b****),** interleukin-6 **(****Figure 7c****)** and tumor necrosis factor-alpha **(****Figure 7d****)** were measured in culture supernatants by ELISA following administration of DC alone or pretreated with ApoB100, IL10 and/or TGFb2 as indicated.

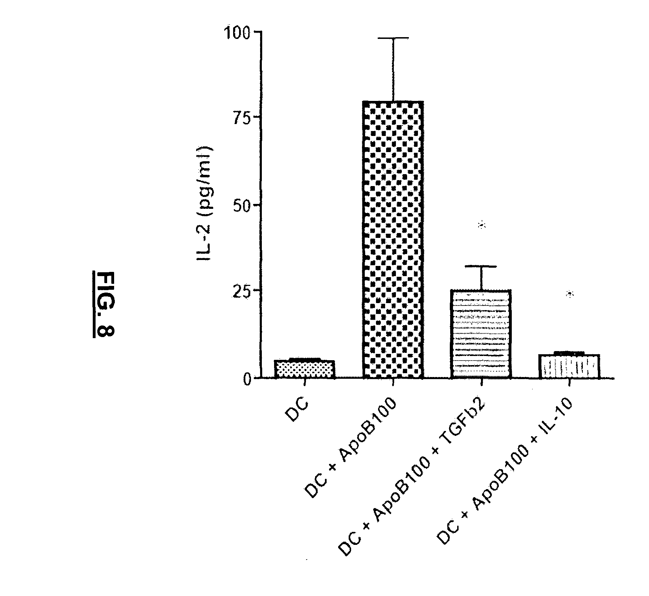

**Figure 8** shows a chart illustrating the effect of tolerogenic DC on antigen-specific activation of the ApoB100 specific T cell hybridoma, 48-5 according to an embodiment herein described. Activation was measured as interleukin-2 secretion into the culture medium and by using ELISA following administration of DC alone or pretreated with ApoB100, IL10 and/or TGFb2 as indicated. Data marked with * indicate significant difference from the group treated with DC+ApoB100, at P<0.05.

### Detailed description

Provided herein are tolerogenic antigen-presenting cells presenting an antigenic portion of an antigen and in particular of an autoantigen, a fragment thereof or a derivative thereof.

The term "tolerogenic", as it is used herein, relates to a substance that can lead to immunological tolerance. In particular, a tolerogenic substance in the sense of the present disclosure comprises any substance that is able, under appropriate conditions identifiable by a skilled person to minimize to the immune response to an antigen.

The term "antigen presenting cells" as used herein indicates immune cells whose main function is to process antigen material and present it on the surface to other cells of the immune system, thus functioning as antigen-presenting cells. Exemplary antigen presenting cells comprise dendritic cells, macrophages, B-cells and additional cells identifiable by a skilled person. The antigen presenting cells (APC) can be of any origin, and in particular human origin. In the present disclosure, reference is often made to dendritic cells (DC) which have the broadest range of antigen presentation as exemplary APC. A skilled person will be able to apply the indications made for the DC to other APC herein described.

The term "antigen", as it is used herein, relates to any substance that, when introduced into the body can stimulate an immune response. Antigens comprise exogenous antigens (antigens that have entered the body from the outside, for example by inhalation, ingestion, or injection) and endogenous antigens or autoantigens (antigens that have been generated within the body). In particular, an "autoantigen" is an antigen that despite being a normal tissue constituent is the target of a humoral or cell-mediated immune response. Exemplary autoantigens comprise collagen type II (arthritis), myelin associated proteins (multiple sclerosis), glutamic acid decarboxylase (type 1 diabetes), and thyrotropin receptors (autoimmune thyroiditis).

The term "antigen-specific" as used indicates an immunitary response, and in particular, immunological tolerance, for a certain antigen which is characterized by a substantially less or no immune response (and in particular, immunological tolerance) for another antigen. Accordingly, an antigen specific tolerogenic cell, specific for one or more autoantigens presented on the tolerogenic cell is able, under appropriate conditions to minimize to the specific immune response to the one or more autoantigens with substantially less or no minimizing effect on the immune response towards other antigens or autoantigens.

The term "presenting" used herein with reference to antigen indicates displaying of at least one antigenic portion of the antigen to responding T cells, in particular performed through binding to an MHC molecule on the surface of an antigen-presenting cell. Accordingly, an antigen presented on a dendritic cell, is able to trigger under the appropriate conditions the one or more reactions that immunogenically characterize the antigen. Typically presentation is performed in outcome of a process by which antigen presenting cells capture an antigen and then enable their recognition by T-cells (herein also load or loading process).

The antigen-specific tolerogenic APC according to the disclosure can be loaded with an autoantigen associated to atherogenesis and/or atherosclerosis.

An exemplary autoantigen associated to atherogenesis and/or atherosclerosis is provided low-density lipoprotein and its constituent protein, ApoB100, are considered to be autoantigens as for example indicated (Hansson GK,Libby P,Nat Rev Immunol 2006;6:508-519).

Additional autoantigens comprise other protein components of a plasma lipoprotein, such as apolipoprotein B48, apolipoprotein E, apolipoprotein Al, apolipoprotein CII or apolipoprotein CIII, heat shock protein-60, human beta2-glycoprotein-I, proteins and protein-derived peptides from cytomegalovirus or herpes simplex virus type 1 or other virus known to be present in atherosclerotic lesions.

In various embodiments, the autoantigen is ApoB100 in various forms. In particular, in an embodiment, the autoantigen of atherosclerosis used in the present disclosure can be ApoB100. In an embodiment, the autoantigen can comprise an LDL particle containing ApoB100, a fragment or peptide component of ApoB100 and/or a derivative thereof. Additional variations are identifiable by a skilled person upon reading of the present disclosure.

The term "ApoB100" as used herein indicates the protein component of LDL apolipoprotein B100 identifiable by a skilled person.

The term "protein" as used herein indicates one or more polypeptides with a particular secondary and tertiary structure that can contain a non-peptide group (e.g. prosthetic group) covalently or non-covalently attached to the polypeptide, and can contain carbohydrates covalently attached to the polypeptide chain (glycosylated proteins).The term polypeptide" as used herein indicates an organic polymer composed of two or more amino acid monomers and/or analogs thereof. The term "polypeptide" includes amino acid polymers of any length including full length proteins and peptides, as well as analogs and fragments thereof. A polypeptide of three or more amino acids is also called an oligopeptide. As used herein the term "amino acid", "amino acidic monomer", or "amino acid residue" refers to any of the twenty naturally occurring amino acids including synthetic amino acids with unnatural side chains and including both D and L optical isomers. The term "amino acid analog" refers to an amino acid in which one or more individual atoms have been replaced, either with a different atom, isotope, or with a different functional group but is otherwise identical to its natural amino acid analog. In an embodiment, antigen-specific tolerogenic dendritic cells herein described present an antigenic portion of ApoB100 or other autoantigen, a fragment thereof or a derivative thereof.

The term "fragment" as used herein indicates a portion of a polypeptide of any length. An antigenic fragment of ApoB100 or another autoantigen is accordingly a portion of apoB-100 or the other autoantigen that presents antigenic properties detectable using methods and techniques identifiable by a skilled person. In particular, a fragment of ApoB100 as used herein indicates any portion of the protein ApoB100 of any length, whether derived by actual fragmentation of ApoB100 or of a derivative thereof, or by chemical and/or genetic recombinant synthesis. A fragment or peptide component of ApoB100 or of another autoantigen has essentially the same antigenic properties as ApoB100 or of the other autoantigen.

Examples of antigenpeptides of ApoB100 are described in Fredrikson GN et al, Identification of immune responses against aldehyde-modified peptide sequences in apoB associated with cardiovascular disease. Arterioscler Thromb Vase Biol 2003;23:872-8 and in Fredrikson GN et al., Inhibition of atherosclerosis in apoE-null mice by immunization with apoB-100 peptide sequences. Arterioscler Thromb Vase Biol 2003;23:879-84. Further exemplary antigenic fragments of ApoB100 comprise peptides described in PCT/SE2001/000570 and PCT/SE2004/001239, or a derivative thereof as will be understood by a skilled person

Additional suitable peptides or fragments can be identified by a skilled person by incubating candidate peptide with an MHCII class molecule and detecting the ability of the candidate peptide to bind to the MHC class II molecule, which can be performed by methods identifiable by a skilled person. In some embodiments, suitable fragments can also be identified from a mixture or library of candidate peptides by introducing it in a T cell activation assay with autoantigen-reactive T-cells (e.g. ApoB100 reactive T-cells) and using a standard method to measure T cell activation. Additional agents can be included in order to improve antigen presentation and/or tolerization, such as cytokines, microbial components, or other bioactive compounds.

The term "derivative" as used herein with reference to a first polypeptide (e.g., apoB-100 or fragment thereof), indicates a second polypeptide that is structurally related to the first polypeptide and is derivable from the first polypeptide by a modification that introduces a feature that is not present in the first polypeptide, while retaining functional properties of the first polypeptide. Accordingly, a derivative polypeptide of an antigenic fragment of apoB-100 or other autoantigen, usually differs from the original polypeptide or portion thereof by modification of the amino acidic sequence that might or might not be associated with an additional function not present in the original polypeptide or portion thereof. A derivative polypeptide of an antigenic fragment of apoB-100 or other autoantigen retains however antigenic properties comparable to the ones described in connection with the original autoantigen or the antigenic fragment thereof. Derivatives suitable in the antigen specific tolerogenic cells herein described can be identified with the same method suitable to identify peptides herein described wherein candidate derivatives are incubated with an MHCII class molecule and related binding detected.

In particular, a derivative of ApoB100 in the sense of the present disclosure comprises at least one epitope of ApoB100 or of a fragment thereof, which are identifiable by a skilled person, In particular, identification of an epitope of ApoB100 can be performed by exposing CD4+ T cells to oligopeptides derived from ApoB100 under antigen-presenting conditions as described above and measuring either DNA synthesis or cytokine secretion by the T cells as indicators of activation. Exemplary derivatives of ApoB100 or of a fragment thereof comprise oxidative forms, and in particular modifications that result in the formation of an aldehyde adduct such as a malondialdehyde or 5-hydroxynonenal derivative on the peptide chain of the antigen. Additional derivatives comprise proteins with modifications such as glycosylation, phosphorylation, adducts derived from reactive agents of LDL lipid peroxidation and additional modifications identifiable by a skilled person that in certain cases can enhance antigenicity.

In an embodiment, ApoB100 can be used as a full-length protein, or as a protein present in a micelle, vesicle or similar preparation identifiable by a skilled person.

In an embodiment, a suitable fragment of ApoB100 or of anotherautoantigen herein described can be a peptide from 1 to about 50 amino acids, and in particular at least from about 9 and up to about 30 amino acids long. Suitable peptides can be synthesized by using a commercial peptide synthesizer, or isolated after proteolytic cleavage of ApoB100 or other autoantigen. Suitable peptides can also be selected by using a bioinformatic approach based on their capacity to bind to human HLA class II molecules. Suitable fragment can also be identified, or validated if identified bioinformatically, by exposing them to a T cell line that recognizes ApoB100 under antigen-presenting conditions, and identify suitable peptides based on the magnitude of T cell activation (using a standard method such as flow cytometry, ELISA of a secreted cytokine, or DNA synthesis). A derivative can be constructed to further improve uptake into the dendritic cell, by fusing or conjugating the peptide to a macromolecule or particle with clustered negative surface charges that makes it amenable to uptake through scavenger receptors.

In an embodiment, antigen-specific tolerogenic dendritic cells of the present disclosure can be loaded with two or more of the autoantigens herein indicated and present one or more of antigenic portions thereof. In an embodiment, these autoantigens are of human origin. In an embodiment, the autoantigens can be recombinant or synthetic molecules with sequences similar or identical to autoantigen occurring in humans. In an alternative or additional approach, a molecule derived from another species can be used to induce the tolerogenic response alone or in combination with a human and/or synthetic molecule.

In various embodiments, antigen-specifictolerogenic dendritic cells according to the present disclosure can be derived by incubating a dendritic cell with ApoB100 a fragment or derivative thereof in combination with the immunosuppressive cytokines to obtain dendritic cells loaded with an antigenic portion of ApoB100, fragment or derivative thereof.

The term "incubating" and "incubated" as used herein indicates a spatial relationship between two items provided for a time and under condition such that at least one of the reciprocal or non reciprocal action or influence between the two items can be exerted. In particular, incubating APC herein described with autoantigen, fragment derivative or precursor comprises any spatial relationship between the APC and the autoantigen, fragment derivative or precursor herein described resulting in uptake of the autoantigen, fragment derivative or precursor, (e.g. through receptors, endocytosis, makropinocytosis or other ways comprising transfection/electroporation/viral infection and other identifiable by a skilled person) and presentation of one or more antigenic portions of the autoantigen on the APC antigenic. In an embodiment, the APC is incubated with a precursor of the autoantigen in the form of nucleic acids of any kind coding for the antigenic portion of ApoB100.In those embodiments, the incubation is performed for a time and under conditions that allow expression and presentation of the antigenic portion of ApoB100 coded by the nucleic acid.

Exemplary procedures for performing incubation are illustrated in the Examples section. A skilled person will be able to identify additional procedure upon reading of the present disclosure.

In an embodiment, the Dendritic Cells (DC) are incubated with ApoB100 or fragment thereof or derivative thereof in combination with an immunosuppressive cytokine, such as a member of the TGF-beta superfamily.

The term "cytokine" as used herein indicates a category of signaling proteins and glycoproteins extensively used in cellular communication that are produced by a wide variety of hematopoietic and non-hematopoietic cell types and can have autocrine, paracrine and endocrine effects, sometimes strongly dependent on the presence of other chemicals An "immunosuppressive cytokine" in the sense of the present description indicates a cytokine capable to reduce the activation or efficacy of the immune system under appropriate conditions identifiable by a skilled person. Immunosuppressive cytokines comprise cytokines of the TGFbeta superfamily and IL-10.

The term "TGF-β superfamily" or "TGF-beta superfamily" indicates a group of immunosuppressive proteins identifiable by a skilled person and that comprises inhibins, activin, anti-müllerian hormone, bone morphogenetic protein, decapentaplegic and Vg-1. Exemplary cytokines of the TGF beta superfamily comprise TGF-β a secreted protein that controls proliferation, cellular differentiation, and other functions in most cells and that exists in three isoforms called TGF-β1, TGF-β2 and TGF-β3.

The term "interleukin 10" or "IL-10" as used herein indicates an immunosuppressive cytokine also known as human cytokine synthesis inhibitory factor (CSIF). In humans IL-10 is encoded by the IL10 gene. IL-10 and other cytokines according to the present disclosure can be isolated from cells or produced according to chemical or recombinant procedures.

In various embodiments one or more autoantigens added together with one or more of the immunosuppressive cytokines herein described, to the cell culture containing DC.

In particular, antigen-specific tolerogenic dendritic cells herein described can be obtained by treatment of dendritic cells (DC) with at least one cytokine selected from the TGF-beta superfamily and/or with interleukin-10 or derivatives thereof. The term "treatment" as used herein with reference to cells and a cytokine or other molecule indicates an incubation performed for a time and under conditions that allow the cytokine to provide at least one of characteristic biological activity on the cell as detectable by techniques identifiable by a skilled person. Treating the APC herein described with an immunosuppressive cytokine can be performed before during or after incubation of the APC with the one or more autoantigen .

In an embodiment, it is possible to use only one cytokine from the TGF-beta superfamily or interleukin-10. In an embodiment, it is also possible to use a combination of one or more cytokine from the TGF-beta superfamily and/or interleukin-10.

Cytokines belonging to the TGF-beta superfamily that can be used according to the present disclosure are TGF-beta 1, TGF-beta 2, TGF-beta 3, TGF-beta 4, TGF-beta 5, inhibins, activins, Mullerian inhibitory substance, bone morphogenetic proteins (BMPs), Growth and Differentiation Factors of the GDF family, and the GDNF family. They include AMH; ARTN; BMP10; BMP15; BMP2; BMP3; BMP4; BMP5; BMP6; BMP7; BMP8A; BMP8B; BMP10; BMP15; GDF1; GDF10; GDF11; GDF15; GDF2; GDF3; GDF3A; GDF5; GDF6; GDF7; GDF8; GDF9; GDNF; INHA; INHBA; INHBB; INHBC; INHBE; LEFTY1; LEFTY2; MSTN; NODAL; NRTN; PSPN; TGFB1; TGFB2; TGFB3.

In an embodiment, the cytokine selected from the TGF-beta superfamily used is TGF-beta-2.

In particular, treatment of APC with at least one cytokine selected from the TGF-beta superfamily and/or with interleukin-10 can be performed by incubating the dendritic cells in the presence the cytokine from the TGF-beta superfamily and/or with interleukin-10. In particular, in an embodiment, an autoantigen, fragment, or derivative incubated in cell culture with IL-10, TGFb2 or another immunosuppressive cytokine or cytokines, and incubated with an antigen under conditions that permit antigen uptake. APC are then matured using LPS or another suitable agent, and can be then either stored or be administered (e.g. injected) into recipients to be treated.

In an embodiment, treatment can be performed with at least one cytokine selected from the transforming growth factor-beta (TGF-beta) superfamily, such as TGF-beta-2, and/or with interleukin-10 in combination with ApoB100, a fragment thereof, or a derivative thereof.

In an embodiment, the dendritic cells or other APC to be used to derive the antigen-specific cells herein described can be isolated from peripheral blood, bone marrow or another hematopoietic or lymphoid organ of the individual to be treated with the tolerogenic APC herein described. In an embodiment APC can be be isolated from a donor individual with histocompatibility antigens that match those of the recipient. In an embodiment, APC can be prepared by differentiating stem cells from the recipient or a matched individual.

In an embodiment, antigen-presenting cell such as a monocyte, a macrophage, or a B lymphocyte can be be isolated from human blood, bone marrow, or lymphoid tissue.

In an embodiment, an APC can be a genetically engineered APC, such as those expressing antigenic portions of antigen ApoB100.

In an embodiment, DCs or other APCs are isolated from bone marrow or blood by standard protocols and stimulated to differentiate by cytokine treatment in cell culture. Common methods to achieve such DC are described in Y.I. Son et al., A novel bulk-culture method for generating mature dendritic cells from mouse bone marrow cells. J Immunol Methods 2002;262:145-157, and by K. Inaba et al. in Curr. Protoc. Immunol.86:3.7.1 -3.7.19, 2009 (John Wiley & Sons, Inc). Additional procedures suitable to perform provide DC are identifiable by a skilled person. In an embodiment, the DC thus provided are incubated with IL-10 and/or a TGF-beta family member. Recombinant IL-10 and TGF-beta2 proteins are commonly used for this purpose but isolated natural proteins may also be used. Conditions suitable for incubation with cytokines and antigen are described by YY. Lan et al. in "Alternatively activated" dendritic cells preferentially secrete IL- 0, expand Foxp3+CD4+ T cells, and induce long-term organ allograft survival in combination with CTLA4-Ig. J. Immunol. 2006;177:5868-5877.

In an embodiment, the tolerogenic dendritic cells are pretreated with interleukin-10.

In an embodiment, purified DCs or other APCs are incubated with optimal concentrations of recombinant IL-10 or TGF-beta2 together with autoantigen in serum-free cell culture medium for 18 hours. After a suitable incubation time (e.g. between about 1 and about 24 hours, in some cases about 4 hours), lipopolysaccharide is added to induce DC maturation. After an amount of time suitable for inducing the desired maturation (e.g. between about 6 and about 48 hours, in some cases about 14 hrs), DC are typically washed in a suitable medium and are ready for administration or other uses (e.g. either injected or kept on ice until time of injection into an individual)

In various embodiments, the antigen-specific tolerogenic DC cells herein described can be used to treat or prevent atherosclerosis or condition associated thereto in an individual.

The term "treatment" used herein with reference to individuals relates to treatment in order to cure or alleviate a disease or a condition. The treatment may either be performed in an acute or in a chronic way.

The term "prevention" used herein relates to treatment in order to prevent the development of a disease or a condition. The preventive treatment is normally used on individuals who have not yet shown any clinical signs of atherosclerosis.

The term "individual" as used herein indicates a single biological organism such as higher animals and in particular vertebrates such as mammals and more particularly human beings.Particular individuals are patients, wherein the term "patient", as it is used herein, relates to any human in need of treatment according to the disclosure.

The term "atherosclerosis" as used herein indicates a chronic inflammatory disease characterized by a massive intimal deposit of cholesterol derived from low density lipoprotein (LDL), and by an inflammatory response against accumulated LDL.

More precisely, atherosclerosis is a chronic disease that causes a thickening of the innermost layer (the intima) of large and medium-sized arteries. It decreases blood flow and may cause thrombosis, ischemia and tissue destruction in organs supplied by the affected vessel. Atherosclerosis is the major cause of cardiovascular disease including myocardial infarction, stroke and peripheral artery disease. It is the major cause of death in the Western world and is predicted to become the leading cause of death in the entire world within two decades.

Research during the last 20 years has shown that atherosclerosis is an inflammatory disease, which develops at sites of cholesterol accumulation in the artery wall.

The importance of adaptive immunity for atherosclerosis is supported by the finding of antibodies and T cells reactive to components of LDL, and further underlined by the finding that severe combined immunodeficiency (SCID) leads to reduced atherosclerosis in hypercholesterolemic experimental animals.

The LDL particle consists of several different molecules including triglycerides, cholesterol esters, phospholipids, and a large protein, apolipoprotein B100 (ApoB100).

The mechanisms of atherosclerosis have been described earlier, for example by G. K. Hansson in N. Engl. J. Med. 2005;352:1685-95, which is incorporated herein by reference in its entirety. Various conditions associated to atherosclerosis which comprises various ischemic cardiovascular diseases, are also indentifiable by a skilled person.

The term "condition" as used herein indicates as usually the physical status of the body of an individual (as a whole or of one or more of its parts) that does not conform to a physical status of the individual (as a whole or of one or more of its parts) that is associated with a state of complete physical, mental and possibly social well-being. Conditions herein described include but are not limited to disorders and diseases wherein the term "disorder" indicates a condition of the living individual that is associated to a functional abnormality of the body or of any of its parts, and the term "disease" indicates a condition of the living individual that impairs normal functioning of the body or of any of its parts and is typically manifested by distinguishing signs and symptoms. Exemplary conditions include but are not limited to injuries, disabilities, disorders (including mental and physical disorders), syndromes, infections, deviant behaviours of the individual and atypical variations of structure and functions of the body of an individual or parts thereof.

The wording "associated to" as used herein with reference to two items indicates a relation between the two items such that the occurrence of a first item is accompanied by the occurrence of the second item, which includes but is not limited to a cause-effect relation and sign/symptoms-disease relation. Exemplary condition associated to atherosclerosis comprise cardiovascular diseases such as coronary heart disease, myocardial infarction, stroke, and/or a peripheral artery disease.

In an embodiment of a method for treatment of atherosclerosis according to the present disclosure, a therapeutically effective amount of the tolerogenic dendritic cells is administered to a patient in need of treatment and/or to an individual in need of prevention of atherosclerosis.

The term "therapeutically effective amount" relates to an amount that will lead to the desired therapeutic effect, i.e. to an effect on atherosclerosis.

In an embodiment, antigen-specific tolerogenic APC can be administered intravenously or by the cutaneous, subcutaneous, nasal, peroral, intramuscular or intraperitoneal route, or by any other route that permits entry of live DC into the organism of the individual to be treated. Additional agents could be used to improve entry and survival of such DC in the recipient individual, and the number of DC administered may depend on the size or body weight of the individual but also on the extent and severity of atherosclerotic disease.

The administration of tolerogenic dendritic cells or other antigen presenting cells presenting an antigenic portion of ApoB100 or a fragment thereof or a derivative thereof, can be performed in dosing intervals between about 1x10⁶ to about 50x10⁶ cells per individual. The exact number of cells administered can be adjusted individually depending on e.g. the extent and severity of atherosclerotic disease. Possible route of administration comprise intravenously, intradermal, cutaneous, subcutaneous, nasal, peroral, intramuscular or intraperitoneal route.

In some embodiments, tolerogenic dendritic cells herein described can be used in the treatment of atherosclerosis performed by dampening the inflammatory response to LDL by injecting mice intravenously with dendritic cells (DC) that had been pulsed with the protein component of LDL apolipoprotein B100 (ApoB100) in combination with the immunosuppressive cytokines IL-10 and/or TGFbeta2 (see Example 2, 3 and 4 below).

In an embodiment, when treated with suppressive cytokines, the APC and in particular, DC produce less of the pro-inflammatory cytokines TNF-alpha, MCP-1 and IL-12. In exemplary procedures carried out with DC and ApoB100, described in detail in the example section, spleen cells from mice injected with tolerogenic DC showed diminished proliferative responses to ApoB100 as compared to cells from mice injected with DC not treated with immunosuppressive cytokines. The dampened immunity was also reflected in the cytokine pattern from the spleen cells with significantly decreased levels of IFN-gamma, IL-5, IL-6, IL-12 and TNF-alpha.

In an embodiment antigen-specific tolerogenic APC herein described are capable under the appropriate conditions identifiable by a skilled person to trigger a specific response. In exemplary procedures carried out with DC and ApoB100, described in detail in the example section, Tolerogenic DC were also shown to induce de novo generation of regulatory T cells. To test the effect of tolerogenic DC on atherosclerosis, disease prone huApoB100tg x Ldlr-/- mice were injected with the ApoB100-pulsed tolerogenic dendritic cells. This led to a very significant reduction of atherosclerotic lesions in the aorta and less infiltration of aortic lesion CD4+ T cells, as compared with untouched controls or those injected with DC treated with antigen or cytokine alone (see Example 2 below). It was thus found that tolerogenic DC pulsed with ApoB100 reduce the autoreactive response against LDL (see Examples 1, 3 , 4 and 5 below). Those results support the Applicants's conclusion of novel possibilities for treatment and/or prevention of atherosclerosis based on the use of the antigen specific tolerogenic cells herein described.

In particular, in some embodiments, tolerogenic APC herein described specific for one or more autoantigens that, alone or in combination with other factors, cause atherogenesis and/or atherosclerosis, can be used in treatment and/or prevention of atherosclerosis and/or a condition associated thereto. In some embodiment, tolerogenic APC specific for fragments and/or derivative of said autoantigens under conditions identifiable by a skilled person upon reading of the present disclosure can also be used in treatment and/or prevention methods for atherosclerosis and/or associated conditions, that are herein described.

For example, in an embodiment, autoantigen specific tolerogenic APC herein described that are specific for ApoB100 or a fragment thereof or a derivative thereof can be used in treatment and/or prevention of atherosclerosis and/or associated conditions. Additional autoantigen specific tolerogenic APC specific for autoantigen causing atherosclerosis comprise , LDL that accumulate in the artery wall during atherosclerosis which are identifiable by a skilled person.

In some embodiments, the antigen-specific tolerogenic dendritic cells herein described are comprised in a composition together with a suitable vehicle (e.g. an excipient) and/or an adjuvant.

The term "vehicle" as used herein indicates any of various media acting usually as solvents, carriers, binders or diluents for the the tolerogenic APC herein described that are comprised in the composition as an active ingredient. In particular, the composition including the tolerogenic APC herein described can be used in one of the methods or systems herein described.

The term excipients as used herein indicates.. an inactive substance used as a carrier for the active ingredients of a composition, (e.g. a medication). Exemplary excipients can also be used to bulk up formulations that contain very potent active ingredients, to allow for convenient and accurate dosage. In addition to their use in the single-dosage quantity, excipients can be used in the manufacturing process to aid in the handling of the active substance concerned. Depending on the route of administration, and form of medication, different excipients may be used that are identifiable by a skilled person.

The term adjuvant as used herein indicates.a pharmacological or immunological agent that modify the effect of other agents (e.g., drugs, vaccines) while having few if any direct effects when given by themselves. They are often included in vaccines to enhance the recipient's immune response to a supplied antigen while keeping the injected foreign material at a minimum. Types of adjuvants include: Immunologic adjuvant that stimulate the immune system and increase the response to a vaccine, without having any specific antigenic effect in itself.

In some embodiments, the adjuvants and vehicles are pharmaceutically acceptable and the resulting composition is a pharmaceutical composition. In some of those embodiments, the pharmaceutical composition is a vaccine.

In an embodiment, the pharmaceutical composition may be formulated as a form suitable for intravenous injection, but also as, cutaneous, subcutaneous, nasal, peroral, intramuscular or intraperitoneal administration. In some cases, nasal or peroral administration may be preferred. Regardless of the route of administration, the pharmaceutical composition according to the disclosure is formulated into pharmaceutically acceptable dosage forms by conventional methods. The amount of the active ingredient will vary depending on the patient to be treated, due to factors such as age, sex, weight etc, and on the route of administration as well as on other factors known to the skilled person.

The disclosure is further illustrated below in the following Examples.

### Examples

The tolerogenic dendritic cells, and related compositions, methods and systems herein described are further illustrated in the following examples, which are provided by way of illustration and are not intended to be limiting.

In particular, the following examples illustrate exemplary methods and systems are based on tolerogenic DC cells presenting antigenic portions of ApoB100 prepared by pulsing. A person skilled in the art will appreciate the applicability of the features described in detail for ApoB100 performed with a different autoantigen fragment thereof or derivative thereof, according to the exemplified methods and systems or other methods and systems according to the present disclosure.

In particular, in the following examples, the following materials and methods were used.

### Preparation of ApoB100

LDL (d = 1.019 - 1.063 g/mL) was isolated by ultracentrifugation from pooled plasma of healthy donors, as described (Havel R et al., J Clin Invest 34:1345-1353, 1955). After isolation, LDL was dialyzed extensively against PBS. One millimolar EDTA was added to an aliquot of LDL to generate unmodified LDL. ApoB100 was obtained as described (Wessel D and Flugge Ul, Anal Biochem 138:141-143,1984), using minor modifications. Briefly, 0.4 ml methanol, 0.1 ml chloroform, and 0.3 ml water were added to 0.1 ml of LDL (1 mg/mL); the suspension was then vortexed and centrifuged at 9000 x g for 1 min. The upper phase was removed and 0.3 ml of methanol added to the lower phase and interphase with precipitated protein, which was mixed again and centrifuged at 9000 x g for 2 min to pellet the protein.

To obtain soluble and pure ApoB100, the protein pellet was resuspended in a minimum volume of 10% SDS (Bio-Rad Laboratories, Hercules, CA, USA) until it solubilized. These preparations first were filtered on a PD-10 column (GE Healthcare, Uppsala, Sweden) to remove excess SDS. They were then purified on a Superdex-200 size-exclusion column (0.5 mL/min, in Tris-HCl, pH 7.4). The first peak that contained ApoB100 was collected, and the extra peaks containing contaminant protein were discarded.

ApoB100 preparations were greater than 90% pure, as evaluated in a second injection into a Superdex-200 column (GE Healthcare, Uppsala, Sweden). Finally, protein concentration was determined by Bradford assay (Bio-Rad Laboratories, Hercules, CA, USA).

### Isolation and loading of dendritic cells

Dendritic cells (DC) were generated by a method adopted from Son et al. (J Immunol Methods. 2002;262:145-57). Femurs and tibias were dissected from mice and the ends of the bones were cut with scissors. The bone marrow was flushed from the bones with DMEM (Gibco) supplemented with 10% FCS. The collected bone marrow was meshed through a 100µm dispenser (Falcon) and centrifuged. The bone marrow cells were depleted of red blood cells by incubating with ACK-lysing buffer and cultured for 8 days in tissue culture dishes (100mmØ) (1x107cells/10ml/dish) in 37°C and 7,5% CO2. The culture medium used was DMEM supplemented with 10% FCS, 50U/ml penicillin, 50µg/ml streptomycin, 2mM L-glutamine, 1 mM sodium pyruvate, 10ng/ml GM-CSF (Peprotech) and 10ng/ml IL-4 (Peprotech). Every 2-3 days 1/2 of the medium was replaced with fresh medium and cytokines. Purification of DC from the differentiated bone marrow cells was performed using a CD11c magnetic cell sorting kit (Miltenyi Biotech) according to the manufacturers' instructions.

For cell transfer experiments Applicants used a slightly modified version of previously published protocols (J. Immunol. 2006;177:5868-5877 and J. Immunol. 2004;172:1991-1995), briefly the purified DC were incubated at a density of approximately 1x10⁶ cells/ml in tissue culture dishes (60mmØ) with or without 100µg/ml ApoB100 and with either IL-10 (30ng/ml) or TGFβ2 (5ng/ml) in serum-free DMEM medium containing 1:100 BD ITS+ Premix (BD Biosciences, Franklin Lakes, NJ, USA), 1 mg/mL BSA (Sigma-Aldrich, St. Louis, MO, USA), 10 mmol/L HEPES (Gibco Invitrogen, Carlsbad, CA, USA), 1 mmol/L Na pyruvate (Gibco Invitrogen, Carlsbad, CA, USA), 1 mmol/L nonessential amino acids (Sigma-Aldrich, St. Louis, MO, USA), and 50 µg/mL gentamycin sulfate (Sigma-Aldrich, St. Louis, MO, USA), at 37°C in a humid 5% CO2 atmosphere. After 4 hours of culture LPS (0,1 ng) was added and the cells were further pulsed for another 14 hours. After pulsing, the cells were washed in DMEM and kept on ice until injected. For measuring cytokine release into the supernatant, the purified DC were pulsed in 96-well plates, 4x105 cells /well.

### Incubation of dendritic cells

Purified DC can be incubated in tissue culture dishes with IL-10 or TGF-β2 at an optimal concentration determined by prior experiments, with or without 100µg/ml ApoB100 in serum-free DMEM medium containing 1:100 BD ITS+ Premix (BD Biosciences, Franklin Lakes, NJ, USA), 1 mg/mL BSA (Sigma-Aldrich, St.Louis, MO, USA), 10 mmol/L HEPES (Gibco Invitrogen, Carlsbad, CA, USA), 1 mmol/L Na pyruvate (Gibco Invitrogen, Carlsbad, CA, USA), 1 mmol/L nonessential amino acids (Sigma-Aldrich, St. Louis, MO, USA), and 50 µg/mL gentamycin sulfate (Sigma-Aldrich, St.Louis, MO, USA), at 37°C in a humid 5% CO2 atmosphere. 4 hours later, LPS (0,1ng/ml; titrated to start DC maturation) is added. After another 14 hours, the cells are washed in DMEM, kept on ice and injected into recipients within 1 hour. Variations of the protocol include the use of alternative tolerizing cytokines instead of IL-10 or TGF-β2; the use of different concentrations of cytokines or antigen; use of ApoB100 fragments or derivatives instead of ApoB100; use of intact LDL particles instead of ApoB100; use of alternative cell culture media; use of alternative agents to differentiate and/or activate the DC; an alternative time intervals for the steps in the procedure.

### Animal experiments and evaluation of disease

To study the effect of tolerogenic dendritic cells in disease development, the inventors injected 11-week-old male huB100tg x Ldlr-/- mice (Skalen et al., 2002, Nature 417:750-4) i.v. with 2.5x10⁵ DC pulsed with antigen and/or cytokines. The mice were fed a high-fat diet (0.15% cholesterol), starting 5 days after the immunization until sacrifice 10 weeks later with CO₂. In some experiments the mice were also immunized subcutaneously with 100ug of ApoB100 emulsified with CFA. All experiments were approved by the local ethics committee.

Blood from sacrificed mice was collected by cardiac puncture and vascular perfusion with sterile RNase-free PBS. Thoracic aortas and hearts were dissected and preserved for lesion analysis. One-third of the spleen was saved for cell experiments, one-third was snap-frozen for RNA isolation and one-third was frozen for cryostat sectioning. Inguinal and lumbar lymph nodes, liver and abdominal aortas also were snap-frozen and saved for RNA isolation.

### In vitro cell culture assays

Splenocytes from treated mice were isolated and resuspended. In 96-well plates, 5 x 10⁵ splenocytes were incubated with or without ApoB100 antigen in 200 µL of DMEM supplemented with 2.5% mouse serum, 50U/ml penicillin, 50µg/ml streptomycin, 2mM L-glutamine, 1 mM sodium pyruvate for 72 hours at 37°C in a humid 5% CO₂ atmosphere. One microcurie ³H-thymidine (Sigma-Aldrich, St. Louis, MO, USA) was added after 60 h, and DNA replication was measured with a scintillation counter (Wallac, Turku, Finland). Results are expressed as stimulation index = ((s - c)/c, where s is the cpm of the sample with antigen and c is the cpm of the sample without antigen. Cytokines secreted in the cell culture supernatants after 72 hours were measured with a mouse IFN-gamma ELISA kit (Mabtech, Sweden) and with the cytometric bead array flex set kit (BD Biosciences, CA, USA) for IL-6, TNF-alpha, and IL-5.

In some experiments, 4x10⁵ antigen-pulsed and cytokine-treated DC were incubated with 1 x10⁵ cells of the T cell hybridoma 48.5, which can recognize human ApoB100 in an I-Ab restricted manner and release IL-2 upon activation. This cytokine was measured with mouse IL-2 ELISA kit (R&D systems, USA).

### Statistical analysis

Values are expressed as mean ± standard error of the mean (SEM) unless otherwise indicated. The nonparametric Mann-Whitney U test was used for pairwise comparisons. Differences between groups were considered significant at P values below 0.05.

### Example 1: Tolerogenic DC promote regulatory T cells and have an impaired capability to induce T cell effector responses

DC were prepared from bone marrow of huB100tg x Ldlr-/- mice and maturated by LPS treatment in the presence or absence of IL-10/TGFb2.

In particular, DC were differentiated from mouse bone marrow in the presence of GM-CSF and IL-4 according to a method adapted from Son et al.

A novel bulk-culture method for generating mature dendritic cells from mouse bone marrow cells.J Immunol Methods 262, 145-157 (2002), CD11 c+ DC were purified using magnetic cell sorting and matured with LPS. 2x105 cells were tested for their capability to induce pro-inflammatory cytokines in the presence or absence of IL-10 and/or ApoB100 for 24h. MCP-1 was measured by ELISA analysis of conditioned media from the DC cultures.

The resulting data illustrated in detail by the charts of **Figure 1****,** show secretion of TNFα, MCP-1 and IL-12 were markedly increased after uptake of ApoB100 but significantly repressed upon concomitant treatment with IL- 10/TGFb2 **(****Figure 1****).**

In particular, as demonstrated by the data shown in **Figure 1A** tolerogenic DC have an impaired capability to produce the chemokine monocyte chemotactic protein-1 (MCP-1 ), a chemoattractant for monocytes and T cells. When comparing the 3 graphs, it is evident that secretion of MCP-1 is inhibited most efficiently when DC were exposed to ApoB100 in the presence of IL-10. This is of importance since IL-12 specifically promotes the development of proinflammatory , proatherosclerotic Th1 effector T cells.

Additionally, as shown by the data illustrated in **Figure 1B** DC prepared as described above also have an impaired capability to produce interleukin-12 (IL-12), a cytokine that promotes the differentiation of naive T cells into Th1 effector cells. IL-12 was measured by ELISA analysis of conditioned media.

Furthermore the results illustrated in **Figure 1C** show that DC prepared as described above also have an impaired capability to produce the proinflammatory cytokine tumor necrosis factor-α (TNF- α). The latter was measured by ELISA analysis of conditioned media.

To assess the functional activity of cytokine treated DC, Applicants added such cells to polyclonally activated spleen CD4+ T cells. In particular, 1x10⁴ DC were isolated from mouse bone marrow and purified by magnetic cell sorting for the DC surface protein, CD11c, treated with or without IL-10, and co-incubated for 96h with 4x10⁴ CD4⁺ T cells that had been purified from mouse spleen and activated with anti-CD3.

Therefore, it would be clear to a skilled person that in view of the above data in antigen presenting cells exemplified by the above mentioned DC secretion of TNFα, MCP-1 and IL-12 are markedly increased after uptake of ApoB100 but significantly repressed upon concomitant treatment with IL-10/TGFb2

Further analysis also indicated that this treatment led to significantly reduced IFNg production, and to increased IL-10 production by these T cells as demonstrated by the data shown in details in the illustration of **Figure 2**.

In particular, **Figure 2** shows charts illustrating the ability of tolerogenic DC to inhibit production of interferon-gamma and stimulate de novo generation of regulatory T cells according to an embodiment herein described and the ability of tolerogenic DC to dampen proliferation of activated T cells according to an embodiment herein described.

Flow cytometric analysis illustrated in **Figure 2** (see in part. left column) shows CD4⁺ T cells that were depleted of natural regulatory T cells, stained with CFSE to visualize cell proliferation, co-cultured for 96h and then stained with antibodies to Foxp3. As illustrated in **Figure 2** cell divisions result in reduced CFSE fluorescence. It can be seen that exposure to DC+TGFβ2 **(****Figure 2** bottom graph in the left column) results in the appearance of 33.43% Foxp3+ regulatory T cells (Treg), many of which have undergone several cell divisions. Exposure of T cells to DC+IL-10 **(****Figure 2** middle graph in left column) results in 3.7% Treg, whereas exposure to control DC only resulted in 1.62% Treg, few of which had divided. The center column shows CFSE fluorescence from the same experiment as histograms. Again, it can be seen that CD4+ Treg cells exposed to DC+TGFb2 have divided several times.

The right column of **Figure 2** shows intracellular staining of CD4+ T cells after 96h of co-incubation. Cells were re-stimulated with PMA and lonomycin was added together with Brefeldin A to for the last 5h of culture. CD4+T cells were then permeabilized and stained with anti-IFNγ and anti-IL-10. It can be seen that T cell cultures exposed to DC+TGFβ2 contained 13% IL-10+ cells,whereas those exposed to DC+IL-10 contained 3.88% and those exposed to DC controls only 2.56% IL-10+ cells. IFNγ+ Th1 effector cells were 6.47% of all CD4+ T cells in cultures exposed to DC controls, 1.56% in those exposed to DC + IL-10, and 1.04% in cultures exposed to DC+TGFβ2.

When compared with non-treated DC, cytokine treated DC increased the number of FoxP3+CD4+ T cells among purified spleen CD4+T cells that had been depleted of pre-existing CD4+CD25+ Tregs, suggesting de novo generation **(****Figure 2****).**

IL-10/TGFb2 treated DC promoted significantly less effector T cell proliferation upon polyclonal stimulation than did regular myeloid DC **(****Figure 2****).**

### Example 2: Mice receiving_IL-10- or TGFβ2-treated DC presenting ApoB100 show significantly reduced atherosclerosis with decreased CD4+ T cell infiltration in lesions and reduced systemic inflammation.

Applicants used huB100tg x Ldlr-/- mice as recipients of tolerogenic dendritic cells. These mice express full-length human ApoB100 in the liver and gut, and display humanized lipoprotein profiles. The huB100tg x Ldlr-/- model permits the use of human ApoB100 as antigen. The mice received one I.V. injection of DC that had been pulsed with either a) medium alone; b) ApoB100; c) ApoB100 + TGFβ2; d) ApoB100 + IL-10; or e) IL-10. Finally, f) one group of mice remained untreated. Lesions were measured as described (Nicoletti A et al, J Clin Invest 1998; 102:910-918).

The results shown in **Figure 3****,** indicate a massive decrease in lesion area of the descending thoracic aorta evident and statistically significant for the groups receiving ApoB100 + TGFβ2 or ApoB100 + IL-10 as compared to those groups receiving only antigen or cytokines **(****Figure 3****).**

Immunohistochemical analysis of cryosections from the aortic root showed significantly reduced infiltration of CD4+ T cells in atherosclerotic lesions of mice receiving ApoB100 loaded tolerogenic DC as compared to mice receiving DC pulsed with ApoB100 alone **(****Figure 4****).**

Plasma levels of IFNγ were also significantly decreased in these mice as compared to mice receiving ApoB100-loaded DC **(****Figure 5****).** In particular, the results illustrated in **Figure 5** show the dampening of systemic inflammation associated with reduced atherosclerosis, by decreasing interferon-gamma levels in plasma of mice after injection of ApoB100-specific tolerogenic DC.

### Example 3: In vivo transfer of IL-10- or TGFβ2-treated ApoB100-pulsed DC reduces splenocyte proliferation to ApoB100

T cell populations are known to contain effector T cells reactive to LDL components (Stemme S et al, Proc Natl Acad Sci USA 1995; 92(9):3893-7; Zhou X et al, Arterioscl Thromb Vasc Biol 2006; 26(4):864-70). To test whether this response can be suppressed the inventors transferred DC pulsed with ApoB100 and treated with either TGFβ2 or IL-10 to mice (as above). One week later, these mice were immunized with 100ug of ApoB100 s.c. to boost the cellular response to the protein.

After another week the in vitro proliferative response of splenocytes to ApoB100 was measured. Mice injected with DC pulsed with TGFβ2 or IL-10 and ApoB100 displayed a suppressed proliferative response to ApoB100 when re-stimulated in vitro **(****Figure 6****).** Therefore, DC treatment dampens the autoreactive immune response to ApoB100.

### Example 4: In vivo transfer of IL-10- or TGFβ2-treated ApoB100-pulsed DC dampens pro-inflammatory cytokine responses

To further investigate the effect of tolerogenic DC transfer on the inflammatory properties of immune cells, supernatants of splenocyte cultures from the in vitro ApoB100 re-stimulation experiment (see above) were analyzed with regards to cytokines secreted. Concentrations of IFN-gamma, IL-5, IL-6 and TNF-alpha were significantly reduced in splenocyte cultures of mice injected with DC pulsed with ApoB100 and treated with IL-10, compared to ApoB100-pulse without IL-10 **(****Figure 7****.).**

Splenocytes from mice injected with DC pulsed with ApoB100 and TGFβ2 secreted significantly less IL-5 in response to ApoB100, and showed a trend towards diminished production of IFN-gamma and IL-6 **(****Figure 7****).** This demonstrates that in vivo DC treatment reduces the autoimmune effector response against ApoB100.

### Example 5: DC pulsed with ApoB100 and treated with IL-10 or TGFβ2 can suppress T cell response to ApoB100

LDL reactive T cells are of a phenotype (CD4+, MHC class II restricted) known to recognize protein components (Stemme S 1995; Zhou X 2006, full references given above). The inventors hypothesized that by making the DC tolerogenic with IL-10 or TGFβ2 treatment in the context of ApoB100, the responding T cells would be less activated. When DC, pulsed with ApoB100 and IL-10 or TGFβ2, were incubated with the ApoB100-specific T cell hybridoma 48.5, they suppressed its activation, as reflected in a significantly reduced IL-2 production when compared to responses to DC pulsed with ApoB100 only **(****Figure 8****).** These data suggest that in vivo DC treatment dampens inflammation and disease by directly acting on the ApoB100 specific T cells.

In view of the above data Applicants conclude that tolerogenic antigen-presening cells herein described can provide a preventive and/or therapeutic treatment of the atherosclerosis or of a condition associated thereto in some embodiments even with a single injection and/or with a low dosage which render at least some embodiments of the tolerogenic antigen-presenting cells herein described a surprisingly effective agent for treatment and/or prevention of atherosclerosis and associated conditions.

In summary, in several embodiments, the present disclosure relates to antigen-specific tolerogenic antigen presenting cells presenting antigenic portions of an autoantigen and to related compositions, methods and systems.

The examples set forth above are provided to give those of ordinary skill in the art a complete disclosure and description of how to make and use the embodiments of the compositions, peptides, proteins, methods and systems of the disclosure, and are not intended to limit the scope of what the inventors regard as their disclosure. All patents and publications mentioned in the specification are indicative of the levels of skill of those skilled in the art to which the disclosure pertains.

It is to be understood that the disclosures are not limited to particular compositions or biological systems, which can, of course, vary. It is also to be understood that the terminology used herein is for the purpose of describing particular embodiments only, and is not intended to be limiting. As used in this specification and the appended claims, the singular forms "a," "an," and "the" include plural referents unless the content clearly dictates otherwise. The term "plurality" includes two or more referents unless the content clearly dictates otherwise. The terms "comprises" and "comprising" used herein means including but not limited to." Unless defined otherwise, all technical and scientific terms used herein have the same meaning as commonly understood by one of ordinary skill in the art to which the disclosure pertains.

Although any methods and materials similar or equivalent to those described herein can be used in the practice for testing of the products, methods and system of the present disclosure, exemplary appropriate materials and methods are described herein as examples and for guidance purpose.

A number of embodiments of the disclosure have been described.

## Claims

1. A method of preparing an antigen-specific tolerogenic antigen-presenting cell comprising incubating ApoB-100, or an antigenic fragment thereof, or a derivative polypeptide differing from ApoB-100 or antigenic fragment thereof by modification of the amino acidic sequence wherein the derivative retains antigenic properties comparable to those of ApoB-100 or the antigenic fragment thereof, or a precursor in the form of a nucleic acid coding for an antigenic portion of ApoB-100; and an immunosuppressive cytokine selected from at least one cytokine of the transforming growth factor-beta (TGF-beta) superfamily and/or interleukin-10 (IL-10) with an antigen-presenting cell, wherein the antigen-presenting cell is treated with the immunosuppressive cytokine before, during, or after the incubation.

2. The method according to claim 1 wherein the derivative of ApoB-100 or antigenic fragment thereof comprises at least one epitope of ApoB-100 or of the fragment thereof.

3. The method according to claim 1 or claim 2 wherein the derivative of ApoB-100 or antigenic fragment thereof is selected from derivatives comprising oxidative forms, including modifications that result in the formation of an aldehyde adduct such as a malondialdhedye or 5-hydroxynonenal derivative on the peptide chain of the antigen, and derivatives comprising proteins with modifications such as glycosylation, phosphorylation, adducts derived from reactive agents of LDL lipid peroxidation or additional modifications that can enhance antigenicity.

4. The method according to any one of claims 1 to 3, wherein the antigen presenting cell is isolated from peripheral blood, bone marrow or another hematopoietic or lymphoid organ.

5. The method according to any one of claims 1 to 3, wherein the at least one cytokine comprises TGF-beta-2 and/or IL-10.

6. The method according to any one of claims 1 to 5, wherein the incubating is performed by pulsing the antigen-presenting cell with the ApoB-100, the fragment thereof, or the derivative thereof.

7. The method according to any one of claims 1 to 6, wherein the incubating is performed by transfecting or electroporating a nucleic acid coding for the ApoB-100 or portion thereof, the transfecting or electroporating resulting in the expression of the ApoB-100 or portion thereof.

8. An antigen-specific tolerogenic antigen-presenting cell produced by the method of any one of claims 1 to 7 for use in the treatment of a disease.

9. An antigen-specific tolerogenic antigen-presenting cell produced by the method of any one of claims 1 to 7 for use in the treatment and/or prevention of atherosclerosis or of a cardiovascular disease such as coronary heart disease, myocardial infarction, stroke and/or peripheral artery disease in an individual.

10. The antigen-specific tolerogenic antigen-presenting cell for use according to claim 9, wherein the treatment and/or prevention is performed by administering to the individual an effective amount of the tolerogenic antigen-presenting cell.

11. The antigen-specific tolerogenic antigen-presenting cell for use according to claim 10, wherein the effective amount is between 1 x 10⁶ to 50 x 10⁶ antigen-specific tolerogenic antigen-presenting cells.

12. The antigen-specific tolerogenic antigen-presenting cell for use according to any one of claims 10 and 11, wherein the administering is performed in dosing intervals.

13. The antigen-specific tolerogenic antigen-presenting cell for use according to any one of claims 10 to 12, wherein the administering is performed by intradermal, cutaneous, subcutaneous, nasal, peroral, intramuscular, intravenous, or intraperitoneal route.

14. The antigen-specific tolerogenic antigen-presenting cell for use according to any one of claims 9 to 13, wherein the cardiovascular disease is coronary heart disease, myocardial infarction, stroke, and/or a peripheral artery disease.

15. A pharmaceutical composition for use in the treatment and/or prevention of atherosclerosis or of a cardiovascular disease such as coronary heart disease, myocardial infarction, stroke and/or peripheral artery disease in an individual comprising an antigen-specific tolerogenic antigen-presenting cell produced according to the method of any one of claims 1 to 7 and a pharmaceutically acceptable vehicle.

16. The antigen-specific tolerogenic antigen-presenting cell for use according to any one of claims 8 to 14, wherein the antigen-presenting cell is a dendritic cell, a monocyte, a macrophage, or a B lymphocyte.

17. The method according to any one of claims 1 to 7, wherein the antigen-presenting cell is a dendritic cell, a monocyte, a macrophage, or a B lymphocyte.

18. The pharmaceutical composition for use according to claim 15, wherein the antigen-presenting cell is a dendritic cell, a monocyte, a macrophage, or a B lymphocyte.

## Patentansprüche

1. Verfahren zur Herstellung einer antigenspezifischen tolerogenen antigenpräsentierenden Zelle, bei dem man ApoB-100 oder ein antigenes Fragment davon oder ein Polypeptidderivat, das sich von ApoB-100 bzw. dem antigenen Fragment davon durch Modifikation der Aminosäuresequenz unterscheidet, wobei bei dem Derivat antigene Eigenschaften erhalten sind, die mit denen von ApoB-100 bzw. des antigenen Fragments davon vergleichbar sind, oder eine Vorstufe in Form einer für einen antigenen Teil von ApoB-100 codierenden Nukleinsäure; und ein aus wenigstens einem Cytokin der TGF-beta(Transforming Growth Factor-beta)-Superfamilie und/oder Interleukin-10 (IL-10) ausgewähltes immunsuppressives Cytokin mit einer antigenpräsentierenden Zelle inkubiert, wobei die antigenpräsentierende Zelle mit dem immunsuppressiven Cytokin vor, während oder nach der Inkubation behandelt wird.

2. Verfahren nach Anspruch 1, wobei das Derivat von ApoB-100 oder des antigenen Fragments davon wenigstens ein Epitop von ApoB-100 bzw. des Fragments davon umfasst.

3. Verfahren nach Anspruch 1 oder Anspruch 2, wobei das Derivat von ApoB-100 oder des antigenen Fragments davon aus oxidative Formen umfassenden Derivaten, einschließlich Modifikationen, die zur Bildung eines Aldehydaddukts wie eines Malondialdehyd- oder 5-Hydroxynonenal-Derivats auf der Peptidkette des Antigens führen, und Proteine mit Modifikationen wie Glykosylierung, Phosphorylierung, von reaktiven Agentien der LDL-Lipidperoxidation abgeleiteten Addukten oder weiteren Modifikationen, die die Antigenität verbessern können, umfassenden Derivaten ausgewählt ist.

4. Verfahren nach einem der Ansprüche 1 bis 3, wobei die antigenpräsentierende Zelle aus peripherem Blut, Knochenmark oder einem anderen hämatopoietischen oder lymphoiden Organ isoliert wird.

5. Verfahren nach einem der Ansprüche 1 bis 3, wobei das wenigstens eine Cytokin TGF-beta-2 und/oder IL-10 umfasst.

6. Verfahren nach einem der Ansprüche 1 bis 5, wobei das Inkubieren durch Pulsen der antigenpräsentierenden Zelle mit dem ApoB-100, dem Fragment davon oder dem Derivat davon erfolgt.

7. Verfahren nach einem der Ansprüche 1 bis 6, wobei das Inkubieren durch Transfizieren oder Elektroporieren einer für das ApoB-100 oder einen Teil davon codierenden Nukleinsäure erfolgt, wobei das Transfizieren oder Elektroporieren zur Expression des ApoB-100 oder Teils davon führt.

8. Antigenspezifische tolerogene antigenpräsentierende Zelle, hergestellt mit dem Verfahren gemäß einem der Ansprüche 1 bis 7, zur Verwendung bei der Behandlung einer Krankheit.

9. Antigenspezifische tolerogene antigenpräsentierende Zelle, hergestellt mit dem Verfahren gemäß einem der Ansprüche 1 bis 7, zur Verwendung bei der Behandlung und/oder Vorbeugung von Atherosklerose oder einer Herz-Kreislauf-Krankheit wie koronarer Herzkrankheit, Myokardinfarkt, Schlaganfall und/oder peripherer Arterienkrankheit bei einem Individuum.

10. Antigenspezifische tolerogene antigenpräsentierende Zelle zur Verwendung nach Anspruch 9, wobei die Behandlung und/oder Vorbeugung durch Verabreichen einer wirksamen Menge der tolerogenen antigenpräsentierenden Zelle an das Individuum erfolgt.

11. Antigenspezifische tolerogene antigenpräsentierende Zelle zur Verwendung nach Anspruch 10, wobei die wirksame Menge zwischen 1 x 10⁶ und 50 x 10⁶ antigenspezifischen tolerogenen antigenpräsentierenden Zellen liegt.

12. Antigenspezifische tolerogene antigenpräsentierende Zelle zur Verwendung nach einem der Ansprüche 10 und 11, wobei das Verabreichen in Dosierungsintervallen erfolgt.

13. Antigenspezifische tolerogene antigenpräsentierende Zelle zur Verwendung nach einem der Ansprüche 10 bis 12, wobei das Verabreichen über den intradermalen, kutanen, subkutanen, nasalen, peroralen, intramuskulären, intravenösen oder intraperitonealen Weg erfolgt.

14. Antigenspezifische tolerogene antigenpräsentierende Zelle zur Verwendung nach einem der Ansprüche 9 bis 13, wobei es sich bei der Herz-Kreislauf-Krankheit um koronare Herzkrankheit, Myokardinfarkt, Schlaganfall und/oder periphere Arterienkrankheit handelt.

15. Pharmazeutische Zusammensetzung zur Verwendung bei der Behandlung und/oder Vorbeugung von Atherosklerose oder einer Herz-Kreislauf-Krankheit wie koronarer Herzkrankheit, Myokardinfarkt, Schlaganfall und/oder peripherer Arterienkrankheit bei einem Individuum, umfassend eine nach dem Verfahren gemäß einem der Ansprüche 1 bis 7 hergestellte antigenspezifische tolerogene antigenpräsentierende Zelle und ein pharmazeutisch unbedenkliches Vehikel.

16. Antigenspezifische tolerogene antigenpräsentierende Zelle zur Verwendung nach einem der Ansprüche 8 bis 14, wobei es sich bei der antigenpräsentierenden Zelle um eine dendritische Zelle, einen Monozyten, einen Makrophagen oder einen B-Lymphozyten handelt.

17. Verfahren nach einem der Ansprüche 1 bis 7, wobei es sich bei der antigenpräsentierenden Zelle um eine dendritische Zelle, einen Monozyten, einen Makrophagen oder einen B-Lymphozyten handelt.

18. Pharmazeutische Zusammensetzung zur Verwendung nach Anspruch 15, wobei es sich bei der antigenpräsentierenden Zelle um eine dendritische Zelle, einen Monozyten, einen Makrophagen oder einen B-Lymphozyten handelt.

## Revendications

1. Procédé de préparation d'une cellule présentatrice d'antigène tolérogénique spécifique d'antigène comprenant l'incubation d'ApoB-100, ou d'un fragment antigénique de celle-ci, ou un polypeptide dérivé différant d'ApoB-100 ou du fragment antigénique de celle-ci par modification de la séquence d'acides aminés, le dérivé conservant des propriétés antigéniques comparables à celles d'ApoB-100 ou du fragment antigénique de celle-ci, ou d'un précurseur sous la forme d'un acide nucléique codant pour une partie antigénique d'ApoB-100 ; et d'une cytokine immunosuppressive choisie parmi au moins une cytokine de la superfamille des facteurs de croissance transformants bêta (TGF-bêta) et/ou l'interleukine-10 (IL-10) avec une cellule présentatrice d'antigène, dans lequel la cellule présentatrice d'antigène est traitée avec la cytokine immunosuppressive avant, pendant ou après l'incubation.

2. Procédé selon la revendication 1 dans lequel le dérivé d'ApoB-100 ou du fragment antigénique de celle-ci comprend au moins un épitope d'ApoB-100 ou du fragment antigénique de celle-ci.

3. Procédé selon la revendication 1 ou la revendication 2 dans lequel le dérivé d'ApoB-100 ou du fragment antigénique de celle-ci est choisi parmi les dérivés comprenant les formes oxydantes, comprenant des modifications qui résultent en la formation d'un produit d'addition d'aldéhyde tel qu'un dérivé de malondialdéhyde ou de 5-hydroxynonénal sur la chaîne peptidique de l'antigène, et les dérivés comprenant des protéines ayant des modifications telles qu'une glycosylation, une phosphorylation, des produits d'addition dérivés d'agents réactifs de peroxydation de lipides à LDL ou des modifications supplémentaires qui peuvent accroître l'antigénicité.

4. Procédé selon l'une quelconque des revendications 1 à 3, dans lequel la cellule présentatrice d'antigène est isolée à partir de sang périphérique, de moelle osseuse ou d'un autre organe hématopoïétique ou lymphoïde.

5. Procédé selon l'une quelconque des revendications 1 à 3, dans lequel l'au moins une cytokine comprend le TGF-bêta-2 et/ou l'IL-10.

6. Procédé selon l'une quelconque des revendications 1 à 5, dans lequel l'incubation est effectuée par application d'impulsions à la cellule présentatrice d'antigène avec l'ApoB-100, le fragment de celle-ci ou le dérivé de ceux-ci.

7. Procédé selon l'une quelconque des revendications 1 à 6, dans lequel l'incubation est effectuée par transfection ou électroporation d'un acide nucléique codant pour l'ApoB-100 ou une partie de celle-ci, la transfection ou électroporation résultant en l'expression de l'ApoB-100 ou de la partie de celle-ci.

8. Cellule présentatrice d'antigène tolérogénique spécifique d'antigène produite par le procédé selon l'une quelconque des revendications 1 à 7 destinée à être utilisée dans le traitement d'une maladie.

9. Cellule présentatrice d'antigène tolérogénique spécifique d'antigène produite par le procédé selon l'une quelconque des revendications 1 à 7 destinée à être utilisée dans le traitement et/ou la prévention de l'athérosclérose ou d'une maladie cardiovasculaire telle qu'une coronaropathie, un infarctus du myocarde, un accident vasculaire cérébral et/ou une maladie artérielle périphérique chez un individu.

10. Cellule présentatrice d'antigène tolérogénique spécifique d'antigène destinée à être utilisée selon la revendication 9, le traitement et/ou la prévention étant effectués par administration à l'individu d'une quantité efficace de la cellule présentatrice d'antigène tolérogénique.

11. Cellule présentatrice d'antigène tolérogénique spécifique d'antigène destinée à être utilisée selon la revendication 10, la quantité efficace étant comprise entre 1 x 10⁶ et 50 x 10⁶ cellules présentatrices d'antigène tolérogéniques spécifiques d'antigène.

12. Cellule présentatrice d'antigène tolérogénique spécifique d'antigène destinée à être utilisée selon l'une quelconque des revendications 10 et 11, l'administration étant effectuée avec espacement des doses.

13. Cellule présentatrice d'antigène tolérogénique spécifique d'antigène destinée à être utilisée selon l'une quelconque des revendications 10 à 12, l'administration étant effectuée par voie intradermique, cutanée, sous-cutanée, nasale, orale, intramusculaire, intraveineuse ou intrapéritonéale.

14. Cellule présentatrice d'antigène tolérogénique spécifique d'antigène destinée à être utilisée selon l'une quelconque des revendications 9 à 13, la maladie cardiovasculaire étant une coronaropathie, un infarctus du myocarde, un accident vasculaire cérébral et/ou une maladie artérielle périphérique.

15. Composition pharmaceutique destinée à être utilisée dans le traitement et/ou la prévention de l'athérosclérose ou d'une maladie cardiovasculaire telle qu'une coronaropathie, un infarctus du myocarde, un accident vasculaire cérébral et/ou une maladie artérielle périphérique chez un individu comprenant une cellule présentatrice d'antigène tolérogénique spécifique d'antigène produite selon le procédé selon l'une quelconque des revendications 1 à 7 et un véhicule pharmaceutiquement acceptable.

16. Cellule présentatrice d'antigène tolérogénique spécifique d'antigène destinée à être utilisée selon l'une quelconque des revendications 8 à 14, la cellule présentatrice d'antigène étant une cellule dendritique, un monocyte, un macrophage ou un lymphocyte B.

17. Procédé selon l'une quelconque des revendications 1 à 7, dans lequel la cellule présentatrice d'antigène est une cellule dendritique, un monocyte, un macrophage ou un lymphocyte B.

18. Composition pharmaceutique destinée à être utilisée selon la revendication 15, dans laquelle la cellule présentatrice d'antigène est une cellule dendritique, un monocyte, un macrophage ou un lymphocyte B.
